# EUROPEAN PATENT APPLICATION

(11) **EP 1 063 508 A1**
(43) Date of publication of application: **27.12.2000**
(21) Application number: 97949199.0
(22) Date of filing: 19.12.1997
(51) Int. Cl.: G01N 1/10, G01N 33/493, A61B 5/20

(54) **CONTAINER FOR URINE TEST**

(71) Applicant: ITOCHU CORPORATION, Osaka-shi, Osaka 541-77 (JP)
(72) Inventor: MORIMOTO, Ichiro, Suita-shi, Osaka 565 (JP)
(74) Representative: Gossel, Hans K., Dipl.-Ing.
(86) International application number: JP9704744
(87) International publication number: WO9932871

(57) **Abstract**

A cylindrical vessel body (**2**) is formed which has an end opened. Urine taking inlets (**21**, **22**) are formed in the center of the vessel body (**2**). A cover cylinder (**3**) is formed into which the vessel body (**2**) is inserted to block the urine taking inlets (**21**, **22**). A cap (**4**) for blocking the open end of the vessel body (**2**) is attached to the vessel body (**2**). In the outer periphery of the vessel body (**2**), first, second and third vessel protrusions (**24**, **25**, **26**) are formed. The cover cylinder (**3**) is formed with a first cover protrusion (**33**) for engaging with the third vessel protrusion (**26**) at its temporarily retained position and engaging with the first vessel protrusion (**24**) at a full-inserted position of the vessel body (**2**) and a second cover protrusion (**34**) for engaging with the second vessel protrusion (**25**) at the full-inserted position.

## Description

### Technical Filed

The present invention relates to a urine sampling vessel for use in a urinalysis executed in hospitals, examination centers and the like.

### Background Art

In general, a great number of urinalyses are executed in a hospital or an examination center.

As a urine sampling vessel for use in a urinalysis, there is conventionally known one which consists of a cylindrical vessel body formed with a urine taking inlet, a cap detachable from an open end of the vessel body and a cover cylinder formed so that the vessel body can be freely inserted thereinto and pulled out thereof, as disclosed in WO95/09362.

Also, as disclosed in WO95/09362, there is another conventional urine sampling vessel which consists of a cylindrical vessel body, a cap formed detachably from an open end of the vessel body and formed with a urine taking inlet and a cover cylinder formed so that the vessel body can be freely inserted thereinto and pulled out thereof.

In taking urine with these vessels, the cap is attached to the open end of the vessel body and an examinee grips the cap while directing the vessel body downward. Then, the examinee pours his urine toward the urine taking inlet so that the urine is taken into the vessel body through the urine taking inlet. After the urine is taken, the vessel body is inserted into the cover cylinder with a closed end of the vessel body directed downward. The cover cylinder thereby closes the urine taking inlet.

### Problems to be Solved

The above-described urine sampling vessel, in the condition that the urine taking inlet is blocked by the cover cylinder, is put in a vessel holder or the like and then carried to an examination room or the like. However, these conventional urine sampling vessels have a problem that the urinalysis in the examination room is extremely troublesome.

Specifically, one of urinalysis methods using the above urine sampling vessel is carried out by detaching the cap with the vessel body held inserted into the cover cylinder and pouring a reagent or the like into the vessel body through its open end. Another urinalysis method is carried out by pulling out the cover cylinder from the vessel body to which the cap is held attached and pouring a reagent or the like into the vessel body through its urine taking inlet or the like.

However, there is a demand for a urinalysis executable by pouring a reagent or the like into the vessel body through the urine taking inlet with the cover cylinder held on the vessel body. That is, it is required to move the cover cylinder relative to the vessel body to the extent that the urine taking inlet is opened and pour a reagent or the like into the vessel body through the opened urine taking inlet, thereby facilitating a urinalysis.

The conventional urine sampling vessels are provided with no means for temporarily retaining the cover cylinder. Therefore, the vessel body cannot be put into a position inserted to almost half its entire length into the cover cylinder. Accordingly, the conventional urine sampling vessels have a problem of lack of versatility because they cannot be provided for various kinds of urinalysis methods.

Further, in using an examination apparatus, there is a problem that a urinalysis cannot be conducted with the cap and the cover cylinder held attached to the vessel body. In other words, in using an examination apparatus, there is a problem of difficulty in automating the urinalysis because of the necessity for detaching the cap from the vessel body.

Furthermore, in inserting the vessel body or the like into the cover cylinder after taking the urine, the urine stuck to the surface of the vessel body may trickle down the outer surface of the cover cylinder through the open end of the cover cylinder. In this case, there is the possibility that the trickled urine may make the hand dirty.

The present invention has been made in consideration of the above-mentioned respects, and therefore has its object of providing a urine sampling vessel which can facilitate a urinalysis and take urine sanitarily and easily.

### Disclosure of Invention

To attain the above object, a measure taken in the present invention is to allow for temporary retention of a cover cylinder onto a vessel body.

Another measure taken in the present invention is to form a breakable blocking member in a cap.

More specifically, a urine sampling vessel according to a first solution of the present invention includes an elongated cylindrical vessel body having an end closed and the other end opened and a urine taking inlet formed in the center of the vessel body.

The urine sampling vessel further includes a cover cylinder, formed into an elongated cylinder having an end closed and the other end opened and with a diameter slightly larger than the vessel body so that the vessel body is freely inserted thereinto and pulled out thereof, for blocking the urine taking inlet.

Furthermore, the urine sampling vessel includes a cap, formed detachably from the open end of the vessel body, for opening and closing the open end of the vessel body.

In addition, the urine sampling vessel includes a protrusion for retaining the cover cylinder onto the vessel body at least at a temporarily retained position where the urine taking inlet is opened in a condition that the vessel body is inserted into the cover cylinder.

According to the first solution, first in taking urine, the cap and the vessel body are assembled by attaching the cap to the open end of the vessel body. In this condition, an examinee grips the cap while directing the closed end of the vessel body downward and pours his urine toward the urine taking inlet so that the urine flows into the vessel body through the urine taking inlet. As a result, the urine is taken into the vessel body.

After the urine taking is completed, the vessel body is inserted into the cover cylinder with the closed end of the vessel body held directed downward. Through this insertion of the vessel body, the urine taking inlet is blocked by the cover cylinder. Then, the vessel body is carried to an examination room or the like while held inserted into the cover cylinder.

Thereafter, as a form of urinalysis, the cover cylinder is retained onto the vessel body by the protrusion at its temporarily retained position where the urine taking inlet is opened. Namely, the cover cylinder is put into temporarily retained relation onto the vessel body without being fully pulled out thereof. In this condition, a urinalysis is conducted by introducing a reagent or a test paper into the vessel body through the urine taking inlet.

A urine sampling vessel according to a second solution of the invention includes an elongated cylindrical vessel body having an end closed and the other end opened and a urine taking inlet formed in the center of the vessel body.

The urine sampling vessel further includes a cover cylinder, formed into an elongated cylinder having an end closed and the other end opened and with a diameter slightly larger than the vessel body so that the vessel body is freely inserted thereinto and pulled out thereof, for blocking the urine taking inlet.

Furthermore, the urine sampling vessel includes a cap, formed detachably from the open end of the vessel body, for opening and blocking the open end of the vessel body.

In addition, the urine sampling vessel includes a breakable blocking member formed in the cap to block the open end of the vessel body.

According to the second solution, urine is taken in the same manner as done in the first solution. However, in conducting a urinalysis with an examination apparatus, the vessel body is set in the examination apparatus with the cap and the cover cylinder held attached to the vessel body. Then, the blocking member is broken with a stick for introducing a reagent, a stick for taking out a urine sample or another member. Introduction of the reagent or the like or the urine sampling is carried out through an opening created by breaking the blocking member. As a result, the urinalysis is automated.

A urine sampling vessel according to a third solution of the present invention is constructed so that the urine sampling vessel of the second solution further includes a protrusion for retaining the cover cylinder onto the vessel body at least at a temporarily retained position of the cover cylinder where the urine taking inlet is opened in a condition that the vessel body is inserted into the cover cylinder.

According to the third solution, as another form of urinalysis other than using an examination apparatus, the cover cylinder is retained onto the vessel body by the protrusion at its temporarily retained position where the urine taking inlet is opened. Namely, the cover cylinder is put into temporarily retained relation onto the vessel body without being fully pulled out thereof. In this condition, a urinalysis is conducted by introducing a reagent or a test paper into the vessel body through the urine taking inlet.

In a fourth solution of the invention, the protrusion in the first or third solution includes: a plurality of vessel protrusions formed at respective portions of the outer periphery of the vessel body located closer to the closed end of the vessel body than the urine taking inlet and closer to the open end of the vessel body than the urine taking inlet; and a cover protrusion formed in the inner periphery of the cover cylinder to slide over the vessel protrusion when the vessel body is inserted into and pulled out of the cover cylinder.

Further, the cover protrusion and the corresponding vessel protrusion are constructed to engage each other to retain the cover cylinder onto the vessel body at the temporarily retained position of the cover cylinder where the urine taking inlet is opened and at a full-inserted position of the vessel body where the urine taking inlet is blocked,.

According to the fourth solution, the cover cylinder is surely held at its temporarily retained position and the full-inserted position of the vessel body through the engagement between the cover protrusion and the corresponding vessel protrusion.

A urine sampling vessel according to a fifth solution of the invention is constructed so that the cap in the fourth solution is formed with an engagement groove for engaging the open end of the cover cylinder thereto.

Further, the cover protrusion and the corresponding vessel protrusion are formed at respective positions where the open end of the cover cylinder is engageable in close contact with the end surface of the engagement groove of the cap to so that the cover protrusion slides over the vessel protrusion to make an abutment sound between the cover cylinder and the cap when the vessel body is inserted to its full-inserted position into the cover cylinder.

According to the fifth solution, when the vessel body is inserted to its full-inserted position into the cover cylinder, the cover cylinder and the cap make an abutment sound between them. As a result, the full insertion of the vessel body can readily be recognized.

In a sixth solution of the present invention, the vessel protrusions in the fourth solution includes first and second vessel protrusions, disposed at respective portions of the outer periphery of the vessel body closer to the open end thereof than the urine taking inlet and closer to the closed end thereof than the urine taking inlet, for retaining the cover cylinder onto the vessel body at the full-inserted position, and a third vessel protrusion for temporarily retaining the cover cylinder onto the vessel body at the temporarily retained position of the cover cylinder.

Further, the cover protrusion includes a first cover protrusion, located at a portion of the cover cylinder closer to the open end thereof, for engaging the third vessel protrusion at the temporarily retained position of the cover cylinder and engaging the first vessel protrusion at the full-inserted position of the vessel body, and a second cover protrusion, located at a portion of the cover cylinder closer to the closed end thereof, for engaging the second vessel protrusion at the full-inserted position of the vessel body.

According to the sixth solution, upon the engagement between the first vessel protrusion and the first cover protrusion, the second vessel protrusion and the second cover protrusion are engaged together so that the cover cylinder is retained onto the vessel body at the full-inserted position of the vessel body. On the other hand, when the third vessel protrusion and the first cover protrusion are engaged together, the cover cylinder is retained onto the vessel body at its temporarily retained position.

A urine sampling vessel according to a seventh solution of the invention is constructed so that in the first, second or third solution, a plurality of guide grooves extending in a longitudinal direction of the cover cylinder are formed in a portion of the inner periphery of the cover cylinder located closer to the open end thereof to guidedly introduce urine having been stuck to the surface of the vessel body to the inside of the cover cylinder in inserting the vessel body into the cover cylinder.

According to the seventh solution, in inserting the vessel body into the cover cylinder after taking the urine, the urine having been stuck to the surface of the vessel body is guidedly introduced to the inside of the cover cylinder along the guide grooves. As a result, urine taking can be made sanitarily and easily.

In an eighth solution of the invention, the cap in the second solution includes a cylindrical cap body, and a blocking plug formed in the cap body so as to be freely press fitted into and pulled out of the open end of the vessel body. Further, a retention hole is formed at the other end of the cap body for free insertion and detachment of the cover cylinder. And, the external end wall of the blocking plug is formed as the blocking member.

According to the eighth solution, in conducting a urinalysis with an examination apparatus, the external end wall of the blocking plug, which is a blocking member, is broken with a stick for introducing a reagent, a stick for taking out a urine sample or another member. Introduction of the reagent or the like or the urine sampling is carried out through an opening created by breaking the blocking plug.

A urine sampling vessel according to a ninth solution of the invention includes: a cylindrical vessel body having an end closed and the other end opened for containing taken urine; a cap which is formed in a hollow cylinder both end surfaces of which are open, has one end formed so as to be fitted in tight contact onto the open end of the vessel body and includes a blocking member which partitions an internal space thereof; and a urine taking inlet formed in the center of the cap and located closer to the one end of the cap than the blocking member.

Further, the urine sampling vessel includes a cover cylinder, formed into a cylinder having an end closed and the other end opened so that the vessel body and the cap are freely inserted thereinto and pulled out thereof, for blocking the urine taking inlet.

In addition, the urine sampling vessel includes a protrusion for retaining the cover cylinder onto the vessel body at least at a temporarily retained position where the urine taking inlet is opened in a condition that the vessel body is inserted into the cover cylinder.

According to the ninth solution, first in taking urine, the cap and the vessel body are assembled in a urine taking position by attaching the cap to the open end of the vessel body. In this position, an examinee grips the cap while directing the closed end of the vessel body downward, and pours his urine toward the urine taking inlet thereby taking the urine into the vessel body through the urine taking inlet.

After the urine taking is completed, the vessel body is inserted into the cover cylinder with the closed end of the vessel body held directed downward. Through this insertion of the vessel body, the urine taking inlet is blocked by the cover cylinder. Then, the vessel body is carried to an examination room or the like while held inserted into the cover cylinder.

Thereafter, as a form of urinalysis, the cover cylinder is retained onto the vessel body by the protrusion at its temporarily retained position where the urine taking inlet is opened. Namely, the cover cylinder is put into temporarily retained relation onto the vessel body without being fully pulled out thereof. In this condition, a urinalysis is conducted by introducing a reagent or a test paper into the vessel body through the urine taking inlet.

Thereafter, as a form of urinalysis, the cover cylinder is retained onto the vessel body by the engagement between the cover protrusion and the vessel protrusion at its temporarily retained position where the urine taking inlet is opened. Namely, the cover cylinder is put into temporarily retained relation onto the vessel body without being fully pulled out thereof. In this condition, a urinalysis is conducted by introducing a reagent or a test paper into the vessel body through the urine taking inlet.

A urine sampling vessel according to a tenth solution of the invention includes: a cylindrical vessel body having an end closed and the other end opened for containing taken urine; a cap which is formed in a hollow cylinder both end surfaces of which are open, has one end formed so as to be fitted in tight contact onto the open end of the vessel body and includes a breakable blocking member which partitions an internal space thereof; and a urine taking inlet formed in the center of the cap and located closer to the one end of the cap than the blocking member.

Further, the urine sampling vessel includes a cover cylinder, formed into a cylinder having an end closed and the other end opened so that the vessel body and the cap are freely inserted thereinto and pulled out thereof, for blocking the urine taking inlet.

According to the tenth solution, urine is taken in the same manner as done in the ninth solution. However, in conducting a urinalysis with an examination apparatus, the vessel body is set in the examination apparatus with the cap and the cover cylinder held attached to the vessel body. Then, the blocking member is broken with a stick for introducing a reagent, a stick for taking out a urine sample or another member. Introduction of the reagent or the like or the urine sampling is carried out through an opening created by breaking the blocking member. As a result, the urinalysis is automated.

A urine sampling vessel according to an eleventh solution of the present invention is constructed so that the urine sampling vessel of the tenth solution further includes a protrusion for retaining the cover cylinder onto the vessel body at least at a temporarily retained position where the urine taking inlet is opened when the vessel body is inserted into and pulled out of the cover cylinder.

According to the eleventh solution, as another form of urinalysis other than using an examination apparatus, the cover cylinder is retained onto the vessel body by the protrusion at its temporarily retained position where the urine taking inlet is opened. Namely, the cover cylinder is put into temporarily retained relation onto the vessel body without being fully pulled out thereof. In this condition, a urinalysis is conducted by introducing a reagent or a test paper into the vessel body through the urine taking inlet.

In a twelfth solution of the invention, the protrusion in the ninth or eleventh solution includes: a vessel protrusion, formed in the outer periphery of the vessel body, for retaining the cover cylinder onto the vessel body at least at the temporarily retained position of the cover cylinder where the urine taking inlet is opened in a condition that the vessel body is inserted into the cover cylinder; and a cover protrusion, formed in the inner periphery of the cover cylinder to slide over the vessel protrusion when the vessel body is inserted into the cover cylinder, for retaining the cover cylinder onto the vessel body by engaging the vessel protrusion at least at the temporarily retained position of the cover cylinder.

According to the twelfth solution, the cover cylinder is surely held at its temporarily retained position where the urine taking inlet is opened through the engagement between the cover protrusion and the vessel protrusion. Namely, the cover cylinder is put into temporarily retained relation onto the vessel body through the engagement between the cover protrusion and the vessel protrusion. In this condition, a urinalysis is conducted by introducing a reagent or a test paper into the vessel body through the urine taking inlet.

A urine sampling vessel according to a thirteenth solution of the invention is constructed so that the cap in the twelfth solution is formed with a radially enlarged annular member engageable with the open end of the cover cylinder.

Further, the vessel protrusion and the cover protrusion are formed at respective positions where the open end of the cover cylinder is engageable in close contact with the annular member of the cap so that the cover protrusion slides over the vessel protrusion to make an abutment sound between the cover cylinder and the cap when the vessel body is inserted to its full-inserted position into the cover cylinder.

According to the thirteenth solution, when the vessel body is inserted to its full-inserted position into the cover cylinder, the cover cylinder and the cap make an abutment sound between them. As a result, the full insertion of the vessel body can readily be recognized.

In a fourteenth solution of the present invention, the cover protrusion in the twelfth solution includes: a first cover protrusion, located at a portion of the cover cylinder closer to the open end thereof, for engaging the vessel protrusion at the temporarily retained position of the cover cylinder; and a second cover protrusion, located at a portion of the cover cylinder closer to the closed end thereof, for engaging the vessel protrusion at the full-inserted position of the vessel body where the urine taking inlet is blocked.

According to the fourteenth solution, the vessel protrusion and the first cover protrusion are engaged together so that the cover cylinder is retained onto the vessel body at the full-inserted position of the vessel body. On the other hand, when the vessel protrusion and the first cover protrusion are engaged together, the cover cylinder is retained onto the vessel body at its temporarily retained position.

A urine sampling vessel according to a fifteenth solution of the invention is constructed so that in the ninth, tenth or eleventh solution, a plurality of guide grooves extending in a longitudinal direction of the cover cylinder are formed in a portion of the inner periphery of the cover cylinder located closer to the open end thereof to guidedly introduce urine having been stuck to the surfaces of both the vessel body and the cap to the inside of the cover cylinder in inserting the vessel body into the cover cylinder.

According to the fifteenth solution, in inserting the vessel body into the cover cylinder after taking the urine, the urine having been stuck to the surfaces of both the vessel body and the cap is guidedly introduced to the inside of the cover cylinder along the guide grooves. As a result, urine taking can be made sanitarily and easily.

In a urine sampling vessel according to a sixteenth solution of the invention, the vessel body in the ninth solution is constructed so as to be set in an examination apparatus in a condition that the cap is detached with the vessel body held inserted into the cover cylinder or a condition that both the cap and the cover cylinder are detached.

According to the sixteenth solution, a urinalysis can be conducted without the need for transferring the taken urine from the vessel body to the examination apparatus.

In a urine sampling vessel according to a seventeenth solution of the invention, the vessel body in the tenth solution is constructed so as to be set in an examination apparatus with the cap held attached thereto and inserted into the cover cylinder.

According to the seventeenth solution, the vessel body is set in the examination apparatus in a condition that the cap and the cover cylinder are held attached thereto after the urine is taken. Accordingly, a urinalysis can be automatically conducted.

### Effects of the Invention

According to the above solutions, since the protrusion for retaining the cover cylinder onto the vessel body is formed, the cover cylinder can be held at its temporarily retained position onto the vessel body. Accordingly, in a urinalysis after taking the urine, a reagent or a test paper can be inserted into the vessel body in a condition that the cover cylinder is withdrawn to the position that the urine taking inlet is opened. This allows the urinalysis before the disposal or the like of the cover cylinder. As a result, the urinalysis can be simplified.

Further, the inventive urine sampling vessel can be provided for various kinds of urinalysis methods, resulting in increased versatility.

Particularly according to the sixth and fourteenth solutions, since the vessel body is formed with a vessel protrusion and the cover cylinder is formed with a cover protrusion, the cover cylinder can be surely held at its temporarily retained position onto the vessel body through the engagement between the vessel protrusion and the cover protrusion.

According to the fifth and twelfth solutions, when the vessel body is inserted to its full-inserted position into the cover cylinder, an abutment sound is produced between the cover cylinder and the cap. Accordingly, the full insertion of the vessel body can be correctly recognized.

In addition, according to the fifth and twelfth solutions, upon the sealing engagement between the vessel protrusion and the cover protrusion, a seal is also provided between the open end of the cover cylinder and the engagement groove of the cap. As a result, the urine sampling vessel can have a double-sealed structure, thereby preventing leakage of the urine with reliability.

According the second and tenth solutions, since the blocking member provided in the cap is formed breakably, the examination apparatus can automatically form an opening for introducing a reagent or a test paper or taking out a urine sample therethrough. As a result, the urinalysis can be automated.

Particularly according to the sixteenth and seventeenth solutions, the vessel body can be set in the examination apparatus, directly, or with the vessel body held inserted into the cover cylinder, or with the cap and the cover cylinder held attached to the vessel body. As a result, the urinalysis can be surely automated, resulting in providing an extremely sanitary and ease urinalysis.

According to the seventh and fifteenth solutions, since the guide grooves for urine are provided in the inner periphery of the open end of the cover cylinder, in inserting the vessel body into the cover cylinder, the urine having been stuck to the surface of the vessel body and soon can be guidedly introduced to the inside of the cover cylinder with reliability. As a result, the urine can be prevented from trickling down the outer surface of the cover cylinder and urine taking can be made extremely sanitarily and easily.

### Brief Description of the Drawings

Figures **1** through **16** shows a urine sampling vessel according to Embodiment 1 of the present invention.
Figure **1** is a cross-sectional front view partly showing the urine sampling vessel.
Figure **2** is a front view showing a vessel body.
Figure **3** is a plan view showing the vessel body.
Figure **4** is an enlarged front view showing a first full-retaining protrusion.
Figure **5** is an enlarged front view showing a second full-retaining protrusion.
Figure **6** is a front view showing a cover cylinder.
Figure **7** is a cross-sectional view showing the cover cylinder.
Figure **8** is an enlarged cross-sectional view showing an open end portion of the cover cylinder.
Figure **9** is a side view of the cover cylinder when viewed from its open end.
Figure **10** is an enlarged cross-sectional view showing a second cover protrusion.
Figure **11** is a front view showing a cap.
Figure **12** is a cross-sectional view showing the cap.
Figure **13** is a cross-sectional view of the cap taken along the line XIII-XIII of Figure **11**.
Figure **14** is a cross-sectional view showing the urine sampling vessel when the vessel body is inserted partway into the cover cylinder and a urinalysis is conducted.
Figure **15** is a cross-sectional view showing the urine sampling vessel with the vessel body inserted partway into the cover cylinder.
Figure **16** is a cross-sectional view showing the urine sampling vessel during the urine taking.
Figures **17** through **24** shows a urine sampling vessel according to Embodiment 2 of the present invention.

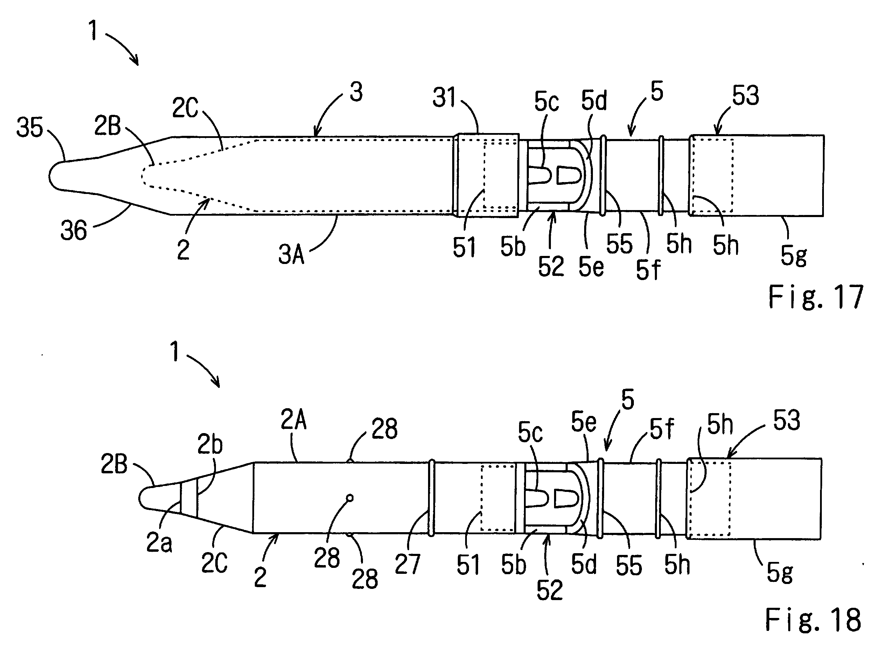

Figure **17** is a front view showing the urine sampling vessel when a vessel body is inserted partway into a cover cylinder and a urinalysis is conducted.
Figure **18** is a front view showing the urine sampling vessel when urine is taken with the cover cylinder detached from the vessel body.

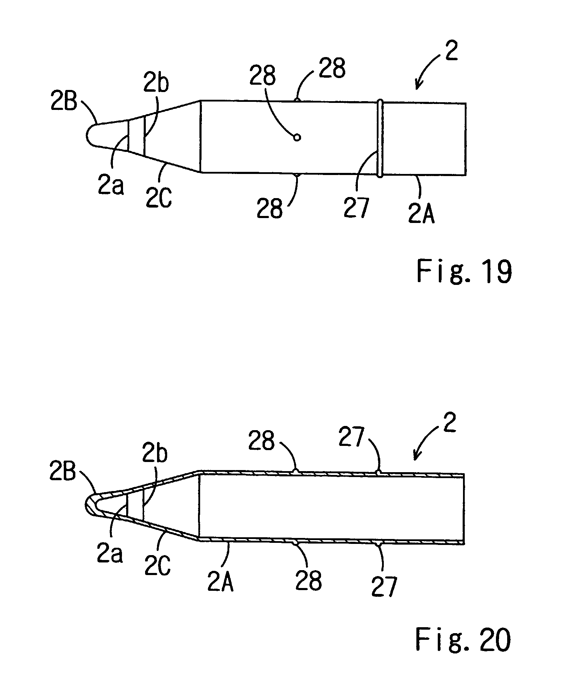

Figure **19** is a front view showing the vessel body.
Figure **20** is a cross-sectional view showing the vessel body.

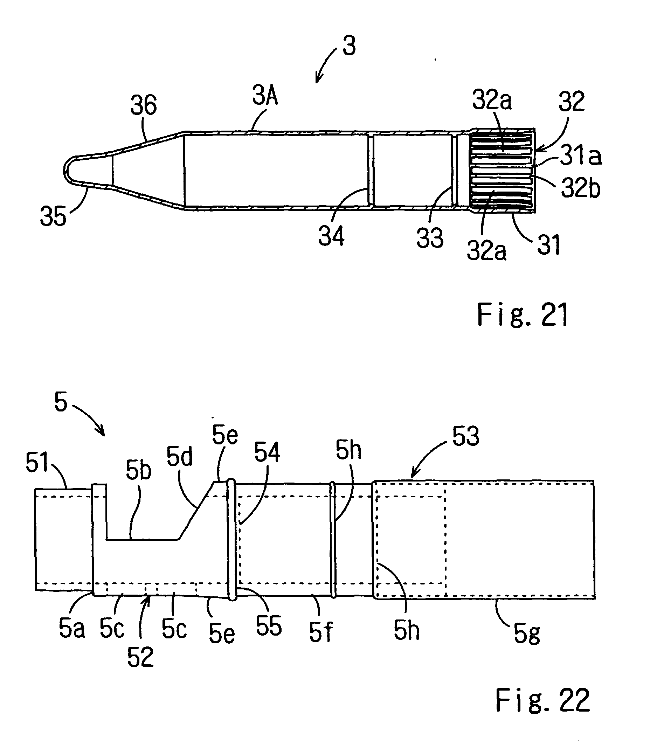

Figure **21** is a cross-sectional view showing the cover cylinder.
Figure **22** is a front view showing a urine taking cap.

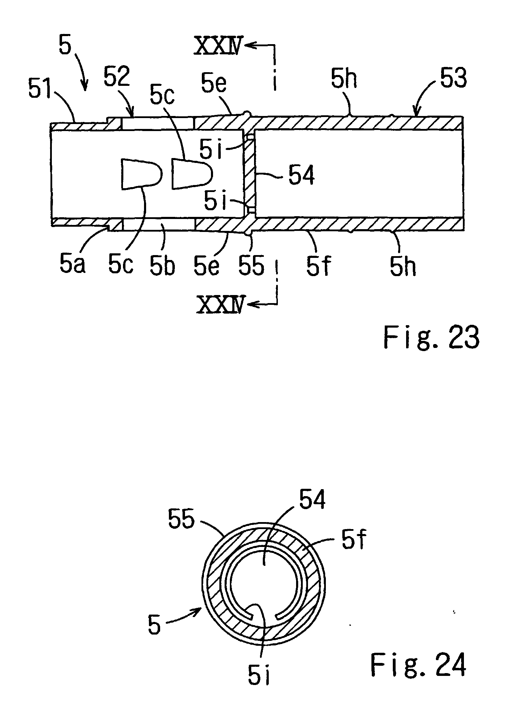

Figure **23** is a cross-sectional view showing the urine taking cap.
Figure **24** is a cross-sectional view of the urine taking cap taken along the line XXIV-XXIV of Figure **23**.

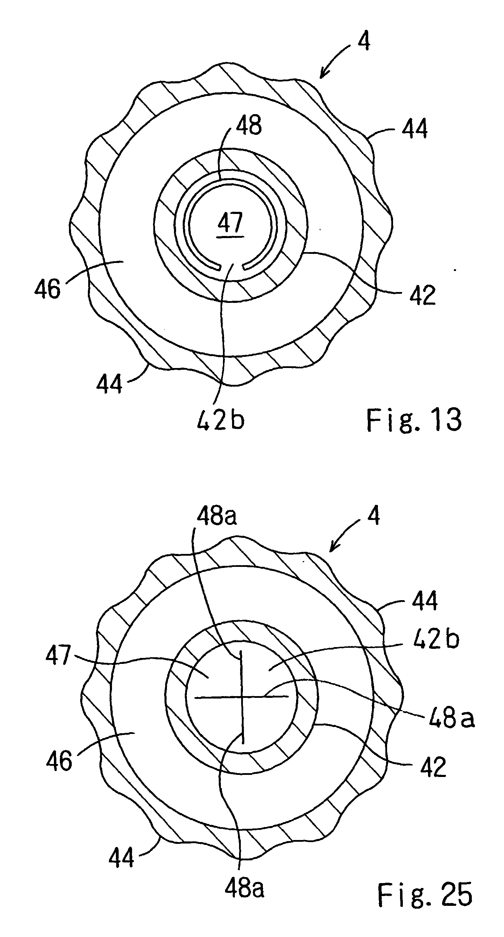

Figure **25** is a cross-sectional view of a cap in another embodiment of the present invention, which corresponds to Figure **13**.

### Best Mode for Carrying Out the Invention

Embodiments of the present invention will be described below in detail with reference to the drawings.

### Embodiment 1

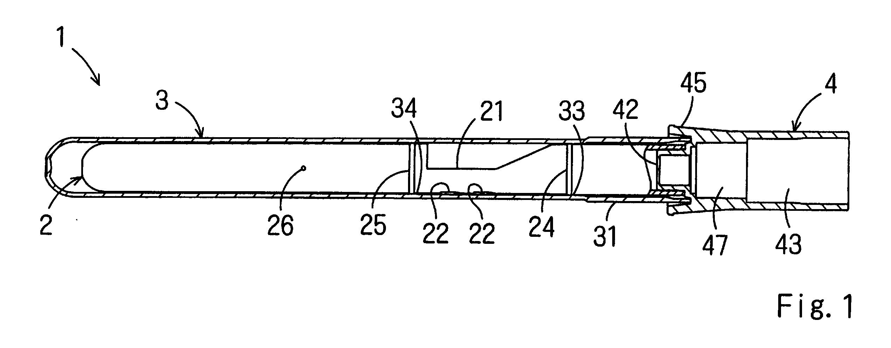

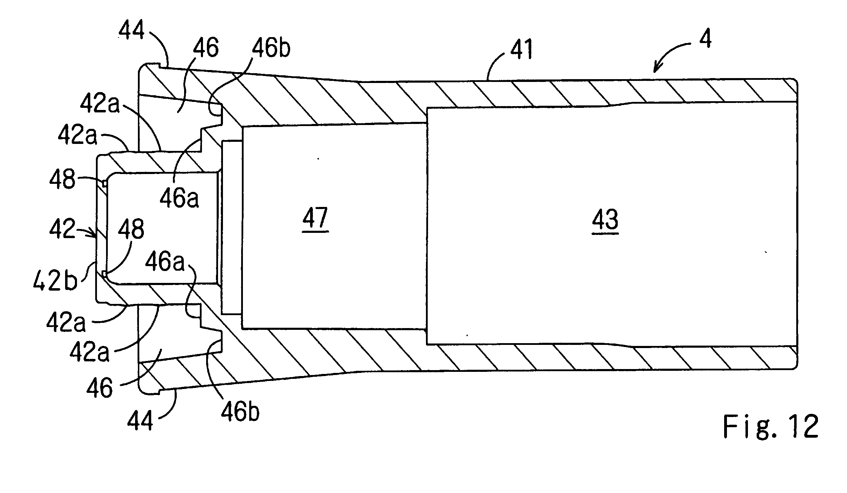

Figures **1** through **16** shows Embodiment 1 of the present invention. As shown in Figure **1**, a urine sampling vessel **1** is a vessel for taking urine in a urinalysis conducted in a hospital or the like.

The urine sampling vessel **1** includes a vessel body **2** for containing urine having been taken, a cover cylinder **3** for covering the vessel body **2**, and a cap **4** detachably attached to the vessel body **2**.

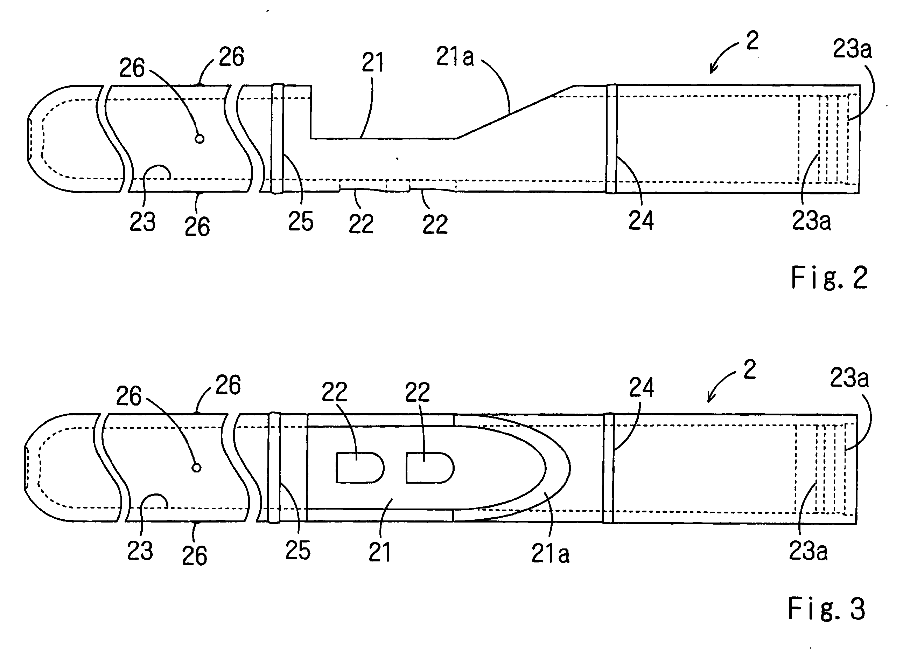

As shown in Figures **2** and **3**, the vessel body **2** is formed in an elongated cylinder having an end closed and the other end opened, and more specifically, a cylinder having an outside diameter of 9.0 mm, an inside diameter of 7.3 mm and a length of 108.5mm. The vessel body **2** is formed of a transparent material such as a synthetic resin.

Furthermore, a first urine taking inlet **21** and second urine taking inlets **22** are formed substantially centrally of the vessel body **2**. Inside of the vessel body **2**, a portion thereof located closer to the closed end than all the urine taking inlets **21**, **22** constitutes a reservoir **23** for urine.

All the urine taking inlets **21**, **22** are openings through which urine flows into the vessel body **2** in taking the urine. And, all the urine taking inlets **21**, **22** are formed into through openings which pass through the vessel body **2** from its outer periphery to inner periphery.

The first urine taking inlet **21** is formed by cutting away part of a circumferentially upper half of the vessel body **2**. The first urine taking inlet **21** is formed in a large opening with an axial length of 22.5 mm, for example. The rear end surface of the first urine taking inlet **21** is formed in an inclined surface **21a** rising toward the open end of the vessel body **2** when viewed in the plan of Figure **3**.

The second urine taking inlets **22** are formed by two in a circumferentially lower half of the vessel body **2** so as to be opposed to the first urine taking inlet **21**. The two second urine taking inlets **22** are formed in series in the axial direction of the vessel body **2**. The second urine taking inlet **22** is formed in a semi-ellipse with an axial length of 4 mm, for example. The reason for providing such second urine taking inlets **22** is that the second urine taking inlets **22** function to release air inside of the vessel body **2** therethrough in taking urine thereby facilitating the urine taking.

In the inner periphery of the open end portion of the vessel body **2**, two annular enlargements **23a** are formed for fixing the cap **4** inserted into the vessel body **2**.

In the outer periphery of the vessel body **2**, vessel protrusions **24**, **25**, **26** of three types as features of the present invention are provided for retaining the cover cylinder **3** onto the vessel body **2**.

The first vessel protrusion **24** is formed at a portion of the vessel body **2** slightly closer to the open end than the urine taking inlets **21**, **22**. The second vessel protrusion **25** is formed at a portion of the vessel body **2** slightly closer to the closed end than the urine taking inlets **21**, **22**. Both the first and second vessel protrusions **24**, **25** are each formed in a ring. In addition, both the first and second vessel protrusions **24**, **25** are constructed so as to retain the cover cylinder **3** onto the vessel body **2** at a full-inserted position of the vessel body **2** where the cover cylinder **3** blocks all the urine taking inlets **21**, **22**.

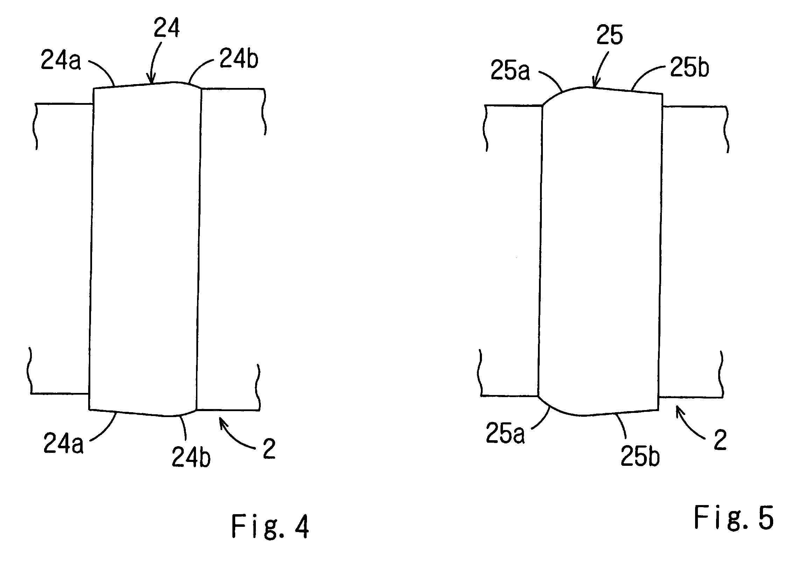

As shown in Figure **4**, the first vessel protrusion **24** consists of a gently inclined surface **24a** located on the closed end side (closer to the urine taking inlets **21**, **22**) and a steeply inclined surface **24b** located on the open end side. The gently inclined surface **24a** continues to the outer periphery of the vessel body **2** through a shoulder. The outside diameter of the first vessel protrusion **24** is set at 9.25 mm, for example.

As shown in Figure **5**, the second vessel protrusion **25** consists of a steeply inclined surface **25a** located on the closed end side and a gently inclined surface **25b** on the open end side (closer to the urine taking inlets **21**, **22**). The gently inclined surface **25b** continues to the outer periphery of the vessel body **2** through a shoulder. The outside diameter of the second vessel protrusion **25** is set at 9.20 mm, for example.

The third vessel protrusions **26** are formed by four each in a semi-sphere and arranged in a circumferential direction of the vessel body **2**. The third vessel protrusions **26** are located at a portion of the vessel body **2** closer to the closed end than the second vessel protrusion **25** and positioned, for example, 39.5 mm away from the closed end. The third vessel protrusions **26** are constructed to retain the cover cylinder **3** onto the vessel body **2** at a temporarily retained position of the cover cylinder **3** where the cover cylinder **3** opens all the urine taking inlets **21**, **22**. The circumscribed circle diameter of the third vessel protrusion **26** is set at 9.15 mm, for example.

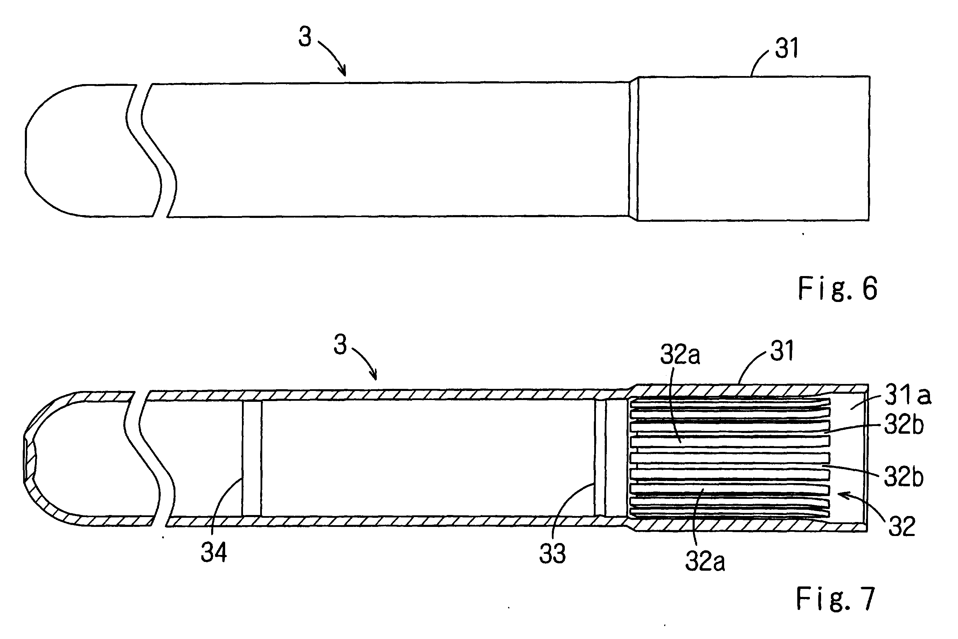

As shown in Figures **6** and **7**, the cover cylinder **3** is formed in an elongated cylinder having an end closed and the other end opened. And, the cover cylinder **3** is formed slightly larger in diameter than the vessel body **2** so that the vessel body **2** can be freely inserted thereinto and pulled out thereof. Specifically, the cover cylinder **3** is formed in a cylinder of 10.8 mm outside diameter, 9.2 mm inside diameter and 116.0 mm length, for example. As shown in Figure **1**, the cover cylinder **3** is formed with such a length as to block all the urine taking inlets **21**, **22** when the vessel body **2** is inserted thereinto. Further, the cover cylinder **3** is formed of a thin, flexible material such as a synthetic resin, and is transparent or translucent. It is to be noted that the cover cylinder **3** may not necessarily be formed of a flexible material and may be slightly thicker.

The cover cylinder **3** has a large-diameter section **31** formed on its open end side. In the inner periphery of the cover cylinder **3**, a urine guide means **32**, a first cover protrusion **33** and a second cover protrusion **34**, by which the present invention is characterized, are formed.

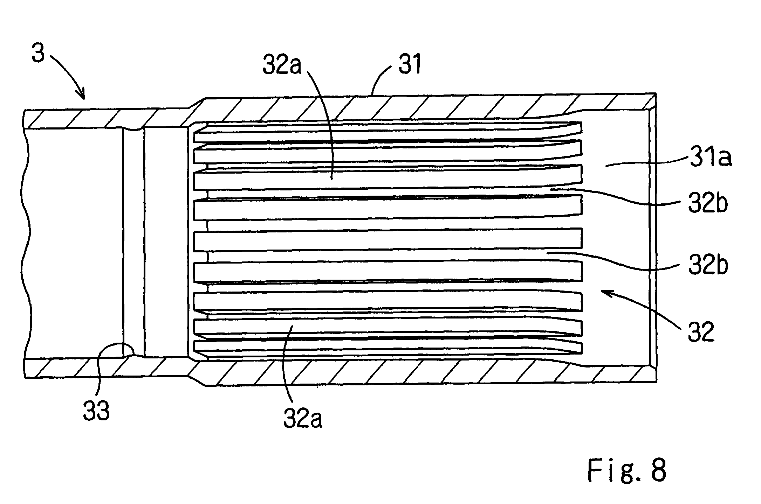

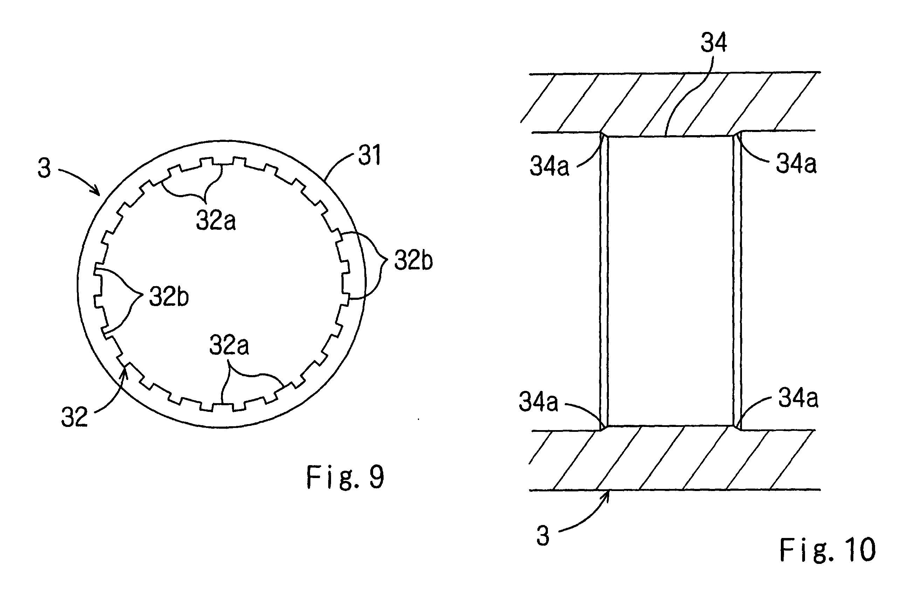

As shown in Figures **8** and **9**, the guide means **32** is formed in the inner periphery of the large-diameter section **31**. The guide means **32** is constructed to guidedly introduce urine having been stuck to the surface of the vessel body **2** to the inside of the cover cylinder **3** when the vessel body **2** is inserted into the cover cylinder **3**. Specifically, the guide means **32** consists of a plurality of guide ribs **32a** extending in a longitudinal direction of the cover cylinder **3** and a plurality of guide grooves **32b** formed between the adjacent guide ribs **32a** to extend in the longitudinal direction of the cover cylinder **3**.

The inscribed circle diameter of the top of the guide rib **32a** is set at 9.5 mm, for example, and is formed slightly larger in diameter than the inner peripheries of the other sections of the cover cylinder **3**. An end portion of the large-diameter section **31** close to the open end has no guide rib **32b** formed in its inner periphery and is formed in a large-diameter opening **31a**. The large-diameter opening **31a** blends into the bottoms of the guide grooves **32b** and is set at an inside diameter of 10.2 mm, for example.

Accordingly, urine stuck to the surface of the vessel body **2** flows through the large-diameter opening **31a** of the large-diameter section **31** into the inside of the cover cylinder **3** along the guide grooves **32b**.

As shown in Figure **8**, the first cover protrusion **33** is formed in the vicinity of the guide means **32** on the open end side of the cover cylinder **3**. The first cover protrusion **33** is formed in a ring of half-round section. The inside diameter of the first cover protrusion **33** is set at 9.1 mm, for example.

As shown in Figure **10**, the second cover protrusion **34** is formed on the closed end side of the cover cylinder **3**. The second cover protrusion **34** is formed in a ring of trapezoidal section so that both ends thereof are formed into slideways **34a**. The inside diameter of the second cover protrusion **34** is set at 9.1 mm, for example.

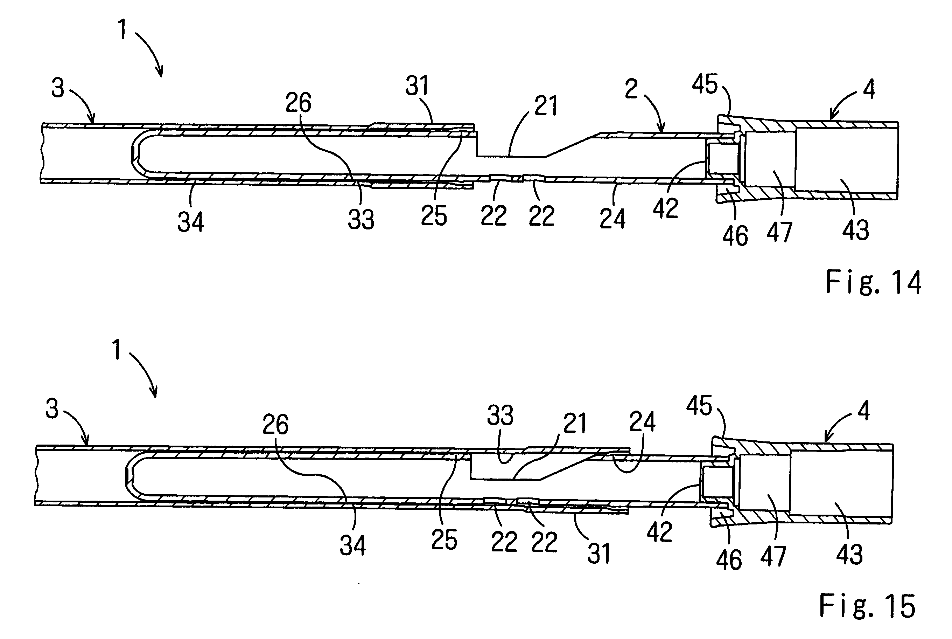

As shown in Figure **14**, the first cover protrusion **33** is constructed to slide over the third vessel protrusions **26** for temporarily retention to engage with them. Through this engagement, the cover cylinder **3** is retained onto the vessel body **2** at its temporarily retained position where the cover cylinder **3** opens all the urine taking inlets **21**, **22**.

As shown in Figure **1**, the first cover protrusion **33** also slides over the first vessel protrusion **24** to engage with it. Concurrently, the second cover protrusion **34** slides over the second vessel protrusion **25** to engage with it. Through these engagements, the cover cylinder **3** is retained onto the vessel body **2** at the full-inserted position of the vessel body **2** where the cover cylinder **3** blocks all the urine taking inlets **21**, **22**.

At the full-inserted position, the end surface of the second cover protrusion **34** engages the shoulder of the second vessel protrusion **25** so that the cover cylinder **3** is retained securely onto the vessel body **2**.

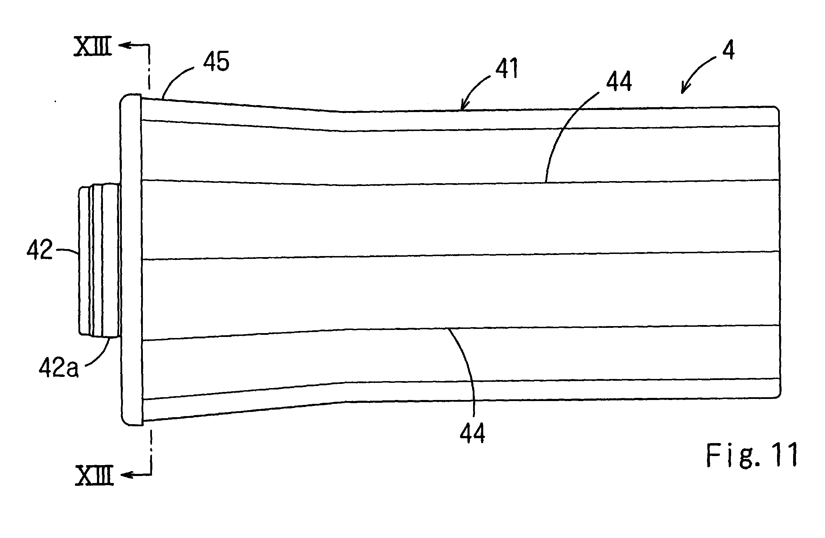

As shown in Figures **11** through **13**, the cap **4** closes the open end of the vessel body **20** and also serves as a grip section in taking urine. And, the cap **4** is constructed so that a cap body **41** is formed with a blocking plug **42** and a retention hole **43**.

The cap body **41** is formed substantially in a cylinder having a length of 34.0 mm, for example. In the outer periphery of the cap body **41**, anti-slip knurls **44** are longitudinally formed. At one end of the cap body **41**, a flange section **45** is formed which gradually enlarges outwardly toward an end surface of the blocking plug **42**.

The blocking plug **42** is formed in a short column so as to be freely press fitted into and pulled out of the open end of the vessel body **20**. In the outer periphery of the blocking plug **42**, two annular enlargements **42a** are formed which are engageable with the two annular enlargements **23a** of the vessel body **2**. The blocking plug **42** is constructed to block the open end of the vessel body **2**.

The retention hole **43** is opened into an remote end surface of the cap body **41** and is formed so that the open end of the cover cylinder **3** can be freely inserted therein and withdrawn therefrom. The retention hole **43** is formed with a depth of 27 mm, for example. A portion of the cap body **41** corresponding to the back of the retention hole **43** is formed thickly in order to securely retain the cover cylinder **3** therein.

The cap body **41** is formed with an engagement groove **46** continuing to the outer periphery of a root end of the blocking plug **42**. The engagement groove **46** is formed to place the open ends of both the vessel body **2** and the cover cylinder **3** therein in a condition that the vessel body **2** is inserted into the cover cylinder **3**. And, the engagement groove **46** is constructed to seal the open ends of the vessel body **2** and the cover cylinder **3** in carrying the vessel.

A radially inner portion of the bottom surface of the engagement groove **46** is formed in a first end surface **46a** of smaller depth against which the open end surface of the vessel body **2** abuts. On the other hand, a radially outer portion of the bottom surface of the engagement groove **46** is formed in a second end surface **46b** of larger depth against which the open end surface of the cover cylinder **3** abuts.

When the cover cylinder **3** is caused to slide onto the vessel body **2** as far as the full-inserted position of the vessel body **2**, the first cover protrusion **33** slides over the first vessel protrusion **24** and the second cover protrusion **34** concurrently slides over the second vessel protrusion **25**. At the time, the first cover protrusion **33** slides down the steeply inclined surface **24b** of the first vessel protrusion **24** and the second cover protrusion **34** concurrently slides down the gently inclined surface **25b** of the second vessel protrusion **25**. A force of this sliding motion of the cover cylinder **3** is received by the cap **4**.

As a result, the open end of the cover cylinder **3** abuts the second end surface **46b** of the engagement groove **46** of the cap **4**. The cover cylinder **3** and the cap **4** are constructed to make an abutment sound to notice the completion of insertion. Further, the open end of the cover cylinder **3** and the second end surface **46b** of the engagement groove **46** are constructed to come into close contact with each other thereby providing a seal therebetween.

In addition, at the full-inserted position, the first cover protrusion **33** and the first vessel protrusion **24** come into close contact with each other thereby providing a seal between the cover cylinder **3** and the vessel body **2**.

Inside of the cap **4**, a hollow section **47** is formed which continues to the retention hole **43** and reaches the external end wall of the blocking plug **42**. The external end wall of the blocking plug **42** is formed in a breakable blocking member **42b** as a feature of the present invention.

Specifically, as shown in Figure **13**, the blocking member **42b** has an arcuate breaking groove **48** formed in the inner surface thereof. The blocking member **42b** is constructed to break along the breaking groove **48** when pushed from the hollow section **47** side.

When the blocking member **42b** is broken, an opening is formed for introducing a reagent or a test paper and taking out a urine sample therethrough. Further, the blocking member **42b** is constructed so that a broken piece thereof remains in the blocking plug **42** with a portion of the blocking member **42b** in which the breaking groove **48** has not been formed.

### Method of Using the Urine Sampling Vessel

Next, a method for using the urine sampling vessel **1** will be described.

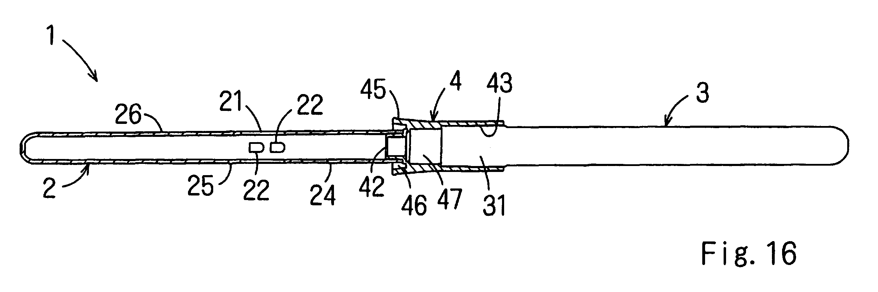

First, in taking urine, the cap **4** is attached with its blocking plug **42** to the open end of the vessel body **2**, and the cover cylinder **3** is inserted at its open end portion into the retention hole **43** of the cap **4** so as to be retained in the cap **4**. Thus, the urine sampling vessel **1** is assembled in a urine taking position as shown in Figure **16**.

In this assembled position of the urine sampling vessel **1**, the examinee grips the cap **4** or the cover cylinder **3** while directing the closed end of the vessel body **2** downward. The examinee then pours urine toward the first urine taking inlet **21** so that the urine flows through the urine taking inlet **21** into the vessel body **2**. The urine is thereby contained in the reservoir **23** of the vessel body **2**. Thus, urine taking is completed.

When the urine taking is completed, the cover cylinder **3** is pulled out of the cap **4** with the closed end of the vessel body **2** held directed downward and the vessel body **2** is inserted into the cover cylinder **3**. At the time, urine is stuck to the surface of the vessel body **2**. The urine flows from the large-diameter opening **31a** down to the inside of the cover cylinder **3** through the guide groove **32b**.

When the vessel body **2** is inserted into the cover cylinder **3**, the first cover protrusion **33** slides over the third vessel protrusions **25** for temporary retention as shown in Figure **14**. Subsequently, the first cover protrusion **33** slides over the second vessel protrusion **25** and the second cover protrusion **34** then slides over the third vessel protrusions **26** as shown in figure **15**. Thereafter, as shown in Figure **1**, the first cover protrusion **33** slides over the first vessel protrusion **24** from the gently inclined surface **24a** and engages with the first vessel protrusion **24**. Concurrently, the second cover protrusion **34** slides over the second vessel protrusion **25** from the steeply inclined surface **25b** and engages the second vessel protrusion **25**. Through these engagements, the cover cylinder **3** is retained onto the vessel body **2** at the full-inserted position of the vessel body **2** where the cover cylinder **3** blocks all the urine taking inlets **21**, **22**.

At this full-inserted position, the first cover protrusion **33** and the first vessel protrusion **24** come into close contact with each other thereby providing a seal between the cover cylinder **3** and the vessel body **2**.

The open end portions of both the cover cylinder **3** and the vessel body **2** are inserted into the engagement groove **46** of the cap **4** so as to be blocked. Particularly, when the cover cylinder **3** is fitted onto the vessel body **2** to the full-inserted position of the vessel body **2**, the first cover protrusion **33** slides over the first vessel protrusion **24** and the second cover protrusion **34** concurrently slides over the second vessel protrusion **25**. At the time, the first cover protrusion **33** slides down the steeply inclined surface **24b** of the first vessel protrusion **24**, and concurrently, the second cover protrusion **34** slides down the gently inclined surface **25b** of the second vessel protrusion **25**. A force of this sliding motion of the cover cylinder **3** is received by the cap **4**.

As a result, the open end of the cover cylinder **3** abuts the second end surface **46b** of the engagement groove **46** of the cap **4**. This produces an abutment sound to notice the completion of insertion of the vessel body **2**. Concurrently, the open end of the cover cylinder **3** and the second end surface **46b** of the engagement groove **46** come into close contact with each other thereby providing a seal therebetween. As a result, a seal is provided between the first cover protrusion **33** and the first vessel protrusion **24**, and another seal is provided between the open end of the cover cylinder **3** and the engagement groove **46** of the cap **4**.

Then, the urine sampling vessel **1** is put in a vessel holder or the like in the condition that the vessel body **2** is inserted into the cover cylinder **3**, and then carried to an examination room or the like.

Thereafter, in conducting a urinalysis, the cap **4** is detached from the urine sampling vessel **1** with the vessel body **2** held inserted into the cover cylinder **3**, and the urinalysis is conducted by inserting a reagent, a test paper or the like into the vessel body **2** through the open end thereof.

In an alternative method, the cover cylinder **3** is pulled out of the vessel body **2** with the cap **4** held attached to the vessel body **2**. Then, a urinalysis is conducted by inserting a reagent, a test paper or the like into the vessel body **2** through the first urine taking inlet **21**.

As a feature of the present invention, the cover cylinder **3** is pulled out for almost a half of its entire length from the vessel body **2** with the cap **4** held attached to the vessel body **2**. Then, as shown in Figure **14**, the first cover protrusion **33** is engaged with the third vessel protrusion **25** for temporary retention. In this condition, the cover cylinder **3** is temporarily retained onto the vessel body **2** at its temporarily retained position. Thereafter, a urinalysis is conducted by inserting a reagent, a test paper or the like into the vessel body **2** through the first urine taking inlet **21**.

As another feature of the present invention, a urinalysis using an examination apparatus is conducted by breaking the blocking member **42b**. Specifically, the urine sampling vessel **1**, which is in the condition that the vessel body **2** is inserted into the cover cylinder **3**, is set in the examination apparatus. Then, a stick for introducing a reagent or a stick for taking out a urine sample, provided in the examination apparatus, is inserted into the hollow section **47** of the cap **4** through the retention hole **43** and the blocking member **42b** is pushed by the stick. Through the push, the blocking member **42b** is broken along the breaking groove **48** to form an opening. Through the opening, a reagent or the like is introduced or a urine sample is taken out.

The broken piece of the blocking member **42b** remains in the blocking plug **42** with a portion of the blocking member **42b** in which the breaking groove **48** has not been formed, and is therefore prevented from falling down to the reservoir **23** of the vessel body **2**.

### Effects of Embodiment 1

According to this embodiment, since the vessel body **2** is formed with three kinds of vessel protrusions **24**, **25** and **26** and the cover cylinder **3** is formed with two cover protrusions **33** and **34**, the cover cylinder **3** can be securely retained at its temporarily retained position onto the vessel body **2**. Accordingly, in a urinalysis after taking the urine, a reagent or a test paper can be inserted into the vessel body **2** in a condition that the cover cylinder **3** is withdrawn from the vessel body **2** down to the position that the urine taking inlets **21**, **22** are opened. This allows the urinalysis before the disposal or the like of the cover cylinder **3**. As a result, the urinalysis can be simplified.

Further, the urine sampling vessel **1** can be provided for various kinds of urinalysis methods, resulting in increased versatility.

Furthermore, when the vessel body **2** is inserted to its full-inserted position into the cover cylinder **3**, an abutment sound is produced between the cover cylinder **3** and the cap **4**. Accordingly, the full insertion of the vessel body **2** can be correctly recognized by simply inserting the vessel body **2** into the cover cylinder **3** until the abutment sound is produced.

Moreover, a seal is provided between the first cover protrusion **33** and the first vessel protrusion **24** and another seal is provided between the open end of the cover cylinder **3** and the engagement groove **46** of the cap **4**. As a result, the urine sampling vessel **1** can have a double-sealed structure, thereby preventing leakage of the urine with reliability.

Further, since the blocking member **42b** provided in the cap **4** is formed breakably, the examination apparatus can automatically form an opening for introducing a reagent or a test paper or taking out a urine sample therethrough. As a result, the urinalysis can be automated.

Furthermore, since the urine guide grooves **32b** are provided in the inner periphery of the open end portion of the cover cylinder **3**, in inserting the vessel body **2** into the cover cylinder **3**, the urine having been stuck to the surface of the vessel body **2** can be guidedly introduced to the inside of the cover cylinder **3** with reliability. As a result, the urine can be prevented from trickling down the outer surface of the cover cylinder **3** and urine taking can be made extremely sanitarily and easily.

### Embodiment 2

Figures **17** through **24** show Embodiment 2 of the present invention. As shown in Figure **17**, a urine sampling vessel **1** is formed larger in diameter than the urine sampling vessel **1** of Embodiment 1.

The urine sampling vessel **1** includes a vessel body **2** for containing urine having been taken, a urine taking cap **5** detachably attached to the vessel body **2**, and a cover cylinder **3** for covering the vessel body **2** and part of the urine taking cap **5**.

As shown in Figures **19** and **20**, the vessel body **2** has an end closed and the other end opened, includes a main section **2A** in the form of an elongated cylinder, and is formed larger in diameter than the vessel body **2** of Embodiment 1. More specifically, the vessel body **2** is formed in a cylinder having an outside diameter of 15.6 through 14.7 mm, an inside diameter of 14.0 mm and an entire length of 87.0 mm. And, the vessel body **2** is formed of a transparent material such as a synthetic resin.

The vessel body **2** has a first diametrically reduced section **2B** and a second diametrically reduced section **2C** which are formed in a tip portion thereof. The first diametrically reduced section **2B** is formed with a taper increasing from the closed end of the tip portion toward the second diametrically reduced section **2C**. The first diametrically reduced section **2B** continues to the second diametrically reduced section **2C** through a first fold. The second diametrically reduced section **2C** is formed with a taper increasing toward the main section **2A**. The second diametrically reduced section **2C** is formed larger in degree of inclination of the taper than the first diametrically reduced section **2B**. The second diametrically reduced section **2C** continues to the main section **2A** through a second fold.

The first fold forms a first index mark **2a**, and a second index mark **2b** is indicated by a micro diametric difference provided at a position of the second diametrically reduced section **2C** located a certain distance away from the first index mark **2a** toward the main section **2A**. The first index mark **2a** and the second index mark **2b** indicate amounts of urine remaining after centrifuging and skimming the taken urine to get a sample for a unitary sediment examination (25 µl; microliter), and are set to display storage amounts of 0.1 cc and 0.2 cc, respectively.

As a feature of the present invention, in the outer periphery of the vessel body **2**, first and second vessel protrusions **27**, **28** are formed for retaining the cover cylinder **3** onto the vessel body **2**.

The first vessel protrusion **27** is formed in a portion of the vessel body **2** closer to the open end of the vessel body **2**. The first vessel protrusion **27** is formed in the shape of a ring. The first vessel protrusion **27** is constructed to retain the cover cylinder **3** onto the vessel body **2** at a full-inserted position of the vessel body **2** where the cover cylinder **3** blocks all urine taking inlets **5b**, **5c** of the urine taking cap **5**. Further, the first vessel protrusion **27** is constructed to retain the cover cylinder **3** onto the vessel body **2** at a temporarily retained position of the cover cylinder **3** where the cover cylinder **3** opens all the urine taking inlets **5b**, **5c** of the urine taking cap **5**.

The second vessel protrusions **28** are formed by four each in a semi-sphere and arranged in a circumferential direction of the vessel body **2**. The second vessel protrusions **28** are located closer to the closed end of the vessel body **2** than the first vessel protrusion **27**. The second vessel protrusions **28** are constructed to come into contact with the inner surface of the cover cylinder **3** to retain the cover cylinder **3** onto the vessel body **2** at an arbitrary position.

As shown in Figures **22** through **24**, the urine taking cap **5** is a cylinder of a colored synthetic resin material and has a fitting cylinder section **51**, a urine taking cylinder section **52** and a grip section **53** continuously provided in order from its inner end closer to the vessel body **2** toward its outer end.

The fitting cylinder section **51** is formed so as to be freely fitted in the open end of the vessel body **2**, and has a hollow whose both ends are opened. More specifically, the fitting cylinder section **51** is formed to have an outside diameter of 14.4 mm and a length of 8 mm, for example.

The urine taking cylinder section **52** is formed in a bottomed cylinder which continues to the fitting cylinder section **51** through a shoulder **5a** and is closed at an outer end (a left-hand end in Figure **23**) thereof. The bottom of the urine taking cylinder section **52** forms a blocking member **54** which closes the outer end of the urine taking cylinder section **52**. The urine taking cylinder section **52** is formed with first and second urine taking inlets **5b**, **5c**. The urine taking cylinder section **52** has almost the same outside diameter as that of the vessel body **2** and has a length of 27 mm, for example.

Both the urine taking inlets **5b**, **5c** are formed in the same manner as done in Embodiment 1. Specifically, the first urine taking inlet **5b** is formed in an opening of an axial length of 15 mm, for example. The first urine taking inlet **5b** has an inclined surface **5d** formed in its rear end surface. The second urine taking inlets **5c** are formed by two each in a semiellipse having an axial length of 4 mm, for example.

The outer periphery of a rear end portion of the urine taking cylinder section **52** (the outer periphery of a portion closer to the grip section **53**) is formed as a tapered surface **5e** whose diameter is increased toward the rear end. The tapered surface **5e** is formed to come into tight contact with the cover cylinder **3** when attached to the vessel body **2** to retain the cover cylinder **3** thereon.

The grip section **53** is formed to continue to the outer end of the urine taking cylinder section **52**, and includes a grip body **5f** and an extension cylinder **5g**. The grip body **5f** is formed in a hollow cylinder having both ends opened.

An annular member **55** for severing the grip section **53** and the taken urine is formed in an enlarged manner in the outer periphery of a connecting portion of the grip section **53** and the urine taking cylinder section **52**.

The bottomed extension cylinder **5g** is slidably fitted onto the grip body **5f** so that the grip section **53** can be axially extended. The grip body **5f** is formed to have a length of 34.5 mm, for example. In the outer periphery of the grip body **5f**, two ribs **5h** are formed at predetermined intervals. The extension cylinder **5g** is formed to have a length of 60 mm, for example.

At the inner end of the extension cylinder **5g**, a slightly inwardly extending flange is formed, though it is not shown. The extension cylinder **5g** is constructed so as to be retained onto the grip body **5f** at two extended positions through the engagement of the flange thereof with one of the ribs **5h**. As a matter of course, the extension cylinder **5g** may be of bottomless structure and may be formed with no inwardly extending flange.

As a feature of the present invention, the blocking member **54** of the urine taking cylinder section **52** is formed breakably. Specifically, as shown in Figure **24**, the blocking member **54** has an arcuate breaking groove **5i** formed in its inner surface. The blocking member **54** is formed to break along the breaking groove **5i** when it is pushed from the grip body **5f** side.

When the blocking member **54** is broken, an opening is formed for introducing a reagent or a test paper or taking out a urine sample therethrough in conducting a urinalysis with an examination apparatus. Further, the blocking member **54** is constructed so that a broken piece thereof remains in the blocking member **54** with a portion thereof in which the breaking groove **5i** has not been formed.

As shown in Figure **21**, the cover cylinder **3** has an end closed and the other end opened and is formed in an elongated cylinder similar to the vessel body **2**. The cover cylinder **3** is formed to allow the vessel body **2** to be freely inserted therein and pulled out thereof. More specifically, the cover cylinder **3** is formed in a cylinder of 16.5 mm outside diameter, 15.9 mm inside diameter and 107 mm length, for example. Further, in a tip portion of the cover cylinder **3**, a first diametrically reduced section **35** and a second diametrically reduced section **36** are formed.

The cover cylinder **3** is formed of a thin, flexible material such as a synthetic resin, and is transparent or translucent. As shown in Figure **17**, the cover cylinder **3** is formed with such a length as to block all the urine taking inlets **5b**, **5c** of the urine taking cap **5** when the vessel body **2** is inserted thereinto. It is to be noted that the cover cylinder **3** may not necessarily be formed of a flexible material and may be slightly thicker.

The cover cylinder **3** has a large-diameter section **31** formed on its open end side. In the inner periphery of the cover cylinder **3**, a urine guide means **32**, a first cover protrusion **33** and a second cover protrusion **34**, by which the present invention is characterized, are formed like Embodiment 1.

The guide means **32** is formed in the inner periphery of the large-diameter section **31**. The guide means **32** is constructed to guidedly introduce urine having been stuck to the surface of the vessel body **2** to the inside of the cover cylinder **3** when the vessel body **2** is inserted into the cover cylinder **3**. Specifically, the guide means **32** consists of a plurality of guide ribs **32a** and a plurality of guide grooves **32b** formed between the adjacent guide ribs **32a**.

It is to be noted that in a condition that the vessel body **2** is inserted into the cover cylinder **3**, the guide ribs **32a** come into tight contact with the tapered surface **5e** of the urine taking cylinder section **52** so that the guide grooves **32b** are blocked by the annular member **55**.

An end portion of the large-diameter section **31** close to the open end has no guide rib **32b** formed in its inner periphery and is formed in a large-diameter opening **31a**. However, in this embodiment, such a large-diameter opening **31a** may not be formed.

The first cover protrusion **33** is formed in the vicinity of the guide means **32** on the open end side of the cover cylinder **3**. The first cover protrusion **33** is formed, for example, in a ring of half-round section like Embodiment 1.

As shown in Figure **21**, the second cover protrusion **34** is formed on the closed end side of the cover cylinder **3**. The second cover protrusion **34** is formed, for example, in a ring of trapezoidal section like Embodiment 1.

The first cover protrusion **33** is constructed to slide over the first vessel protrusion **27** of the vessel body **2** to engage with it. Through this engagement, the cover cylinder **3** is retained onto the vessel body **2** at its temporarily retained position where the cover cylinder **3** opens all the urine taking inlets **5b**, **5c**.

On the other hand, the second cover protrusion **34** is constructed to slide over the first vessel protrusion **27** of the vessel body **2** to engage with it. Through this engagement, the cover cylinder **3** is retained onto the vessel body **2** at the full-inserted position of the vessel body **2** where the cover cylinder **3** blocks all the urine taking inlets **5b**, **5c**.

Further, when the second cover protrusion **34** slides over the first vessel protrusion **27** to the full-inserted position of the vessel body **2**, the second cover protrusion **34** slides down the first vessel protrusion **27**. A force of this sliding motion of the cover cylinder **3** is received by the urine taking cap **5**.

As a result, the open end of the cover cylinder **3** abuts the annular member **55** of the urine taking cap **5**. The cover cylinder **3** and the urine taking cap **5** are constructed to make an abutment sound to notice the completion of insertion. Further, the open end of the cover cylinder **3** and the annular member **55** come into close contact with each other thereby providing a seal between the cover cylinder **3** and the vessel body **2**.

### Method of Using the Urine Sampling Vessel

Next, a method for using the urine sampling vessel **1** will be described.

As shown in Figure **18**, in taking urine, the urine taking cap **5** is attached with its fitting cylinder section **51** to the open end of the vessel body **2**, thereby assembling the urine sampling vessel **1** in a urine taking position. The extension cylinder **5g** of the grip section **53** is slid to regulate the length of the grip section **53** according to the state of use.

In this assembled position of the urine sampling vessel **1**, the examinee grips the grip body **5f** or the slid extension cylinder **5g** of the urine taking cap **5** while directing the closed end of the vessel body **2** downward. The examinee then pours urine toward the first urine taking inlet **5b** so that the urine flows through the urine taking inlet **5b** into the vessel body **2**. The urine is thereby contained in the vessel body **2**. Thus, urine taking is completed.

When the urine taking is completed, the vessel body **2** is inserted into the cover cylinder **3** up to the urine taking cylinder section **52** of the urine taking cap **5** with the closed end of the vessel body **2** held directed downward, and the open end portion of the cover cylinder **3** is brought into tight contact with the tapered surface **5e** to block all the urine taking inlets **5b**, **5c**.

In that case, the extension cylinder **5g** is simultaneously pressed down to the annular member **55** of the urine taking cap **5** to cover the grip body **5f**.

When the vessel body **2** is inserted into the cover cylinder **3**, the first cover protrusion **33** slides over the second vessel protrusions **28** and then the first vessel protrusion **27**. Thereafter, the second cover protrusion **34** slides over the first vessel protrusion **27** and engages with it. Through this engagement, the cover cylinder **3** is retained onto the vessel body **2** at the full-inserted position of the vessel body **2** where the cover cylinder **3** blocks all the urine taking inlets **5b**, **22**.

Further, when the second cover protrusion **34** slides over the first vessel protrusion **27** to the full-inserted position of the vessel body **2**, the open end of the cover cylinder **3** abuts the annular member **55** of the urine taking cap **5** to make an abutment sound, thereby noticing the completion of insertion.

Further, the guide ribs **32a** of the cover cylinder **3** come into tight contact with the tapered surface **5e** of the urine taking cylinder section **52**. Concurrently, the guide grooves **32b** come into close contact with the annular member **55** thereby providing a seal between the cover cylinder **3** and the vessel body **2**.

Then, the urine sampling vessel **1** is put in a vessel holder or the like in the condition that the vessel body **2** and the urine taking cap **5** are inserted into the cover cylinder **3**, and then carried to an examination room or the like.

Thereafter, in conducting a urinalysis, the urine taking cap **5** is detached from the urine sampling vessel **1** with the cover cylinder **3** held attached to the vessel body **2**, and the urinalysis is conducted by setting the vessel body **2** in the examination apparatus.

Alternatively, the urinalysis may be conducted by inserting a reagent, a test paper or the like into the vessel body **2** through the open end or by inserting a reagent, a test paper or the like into the vessel body **2** through the first urine taking inlet **5b** with the urine taking cap **5** held attached to the vessel body **2**.

As a feature of the present invention, the cover cylinder **3** is pulled out for almost a half of its entire length from the vessel body **2** with the urine taking cap **5** held attached to the vessel body **2**. Then, as shown in Figure **18**, the first cover protrusion **33** is engaged with the first vessel protrusion **27**. In this condition, the cover cylinder **3** is temporarily retained onto the vessel body **2** at its temporarily retained position. Thereafter, a urinalysis is conducted by inserting a reagent, a test paper or the like into the vessel body **2** through the first urine taking inlet **5b**.

In the above urinalysis, since the second vessel protrusion **28** of the vessel body **2** comes into contact with the inner surface of the cover cylinder **3**, the cover cylinder **3** may be retained onto the vessel body **2** at an arbitrary position.

As another feature of the present invention, when a urinalysis is conducted with an examination apparatus, the urine sampling vessel **1** is set in the examination apparatus with the urine taking cap **5** and the cover cylinder **3** detached from the vessel body **2** or with the urine taking cap **5** alone detached from the vessel body **2** and held inserted into the cover cylinder **3**.

Alternatively, a urinalysis using an examination apparatus may be conducted by breaking the blocking member **54**. Specifically, the urine sampling vessel **1**, which is in the condition that the vessel body **2** is inserted into the cover cylinder **3**, is set in the examination apparatus. Then, a stick for introducing a reagent or a stick for taking out a urine sample, provided in the examination apparatus, is inserted into the urine taking cap **5** through the grip body **5f** and the blocking member **54** is pushed by the stick. Through the push, the blocking member **54** is broken along the breaking groove **5i** to form an opening. Through the opening, a reagent or the like is introduced or a urine sample is taken out.

The broken piece of the blocking member **54** remains in the urine taking cap **5** with a portion of the blocking member **54** in which the breaking groove **5i** has not been formed, and is therefore prevented from falling down into the vessel body **2**.

### Effects of Embodiment 2

According to this embodiment, since the vessel body **2** is formed with two kinds of vessel protrusions **27** and **28** and the cover cylinder **3** is formed with two cover protrusions **33** and **34**, the cover cylinder **3** can be securely retained at its temporarily retained position onto the vessel body **2** like Embodiment 1. Accordingly, in a urinalysis after taking the urine, a reagent or a test paper can be inserted into the vessel body **2** in a condition that the cover cylinder **3** is withdrawn from the vessel body **2** down to the position that the urine taking inlets **5b**, **5c** are opened. This allows the urinalysis before the disposal or the like of the cover cylinder **3**. As a result, the urinalysis can be simplified.

Further, the urine sampling vessel **1** can be provided for various kinds of urinalysis methods, resulting in increased versatility.

Furthermore, when the vessel body **2** is inserted to its full-inserted position into the cover cylinder **3**, an abutment sound is produced between the cover cylinder **3** and the urine taking cap **5**. Accordingly, the full insertion of the vessel body **2** can be correctly recognized by simply inserting the vessel body **2** into the cover cylinder **3** until the abutment sound is produced.

Moreover, since the blocking member **54** provided in the cap **4** is formed breakably, the examination apparatus can automatically form an opening for introducing a reagent or a test paper or taking out a urine sample therethrough. As a result, the urinalysis can be automated.

Particularly, the vessel body **2** can be set in the examination apparatus, directly, or with the vessel body **2** held inserted into the cover cylinder **3**, or with the urine taking cap **5** and the cover cylinder **3** held attached to the vessel body **2**. As a result, various kinds of urinalyses can be surely automated, resulting in providing an extremely sanitary and ease urinalysis.

Furthermore, since the urine guide grooves **32b** are provided in the inner periphery of the open end portion of the cover cylinder **3**, in inserting the vessel body **2** into the cover cylinder **3**, the urine having been stuck to the surface of the vessel body **2** can be guidedly introduced to the inside of the cover cylinder **3** with reliability. As a result, the urine can be prevented from trickling down the outer surface of the cover cylinder **3** and urine taking can be made extremely sanitarily and easily.

### Other Embodiments

In Embodiment 1, the vessel body **2** is formed with three kinds of vessel protrusions **24**, **25**, **26** and the cover cylinder **3** is formed with two cover protrusions **33**, **34**.

However, the vessel body **2** may be formed with the first vessel protrusion **24** and the third vessel protrusions **26** for temporarily retention and the cover cylinder **3** may be formed with the first cover protrusion **33** alone.

In contrast, the vessel body **2** may be formed with a single vessel protrusion and the cover cylinder **3** may be formed with the first and second cover protrusions **33**, **34**.

Alternatively, the second cover protrusion **34** of the cover cylinder **3** may not necessarily be provided. The shape of the third vessel protrusion **26** for temporarily retention is not limited to a semi-sphere.

In Embodiment 2, the vessel body **2** is formed with two kinds of vessel protrusions **27**, **28** and the cover cylinder **3** is formed with two cover protrusions **33**, **34**.

However, the second vessel protrusion **28** of the vessel body **2** may not necessarily be provided.

Alternatively, the vessel body **2** may be formed with the first and second vessel protrusions **27** and **28** and the cover cylinder **3** may be formed with a single cover protrusion, so that the cover cylinder **3** can be retained at the full-inserted position and the temporarily retained position.

In other words, it is essential in the present invention only that the cover cylinder **3** can be retained at least at the full-inserted position and the temporarily retained position.

The guide means **32** in Embodiment 2 may not necessarily be provided. In this case, it is not necessary to form the large-diameter section **31** in the cover cylinder **3**, and the open end portion of the cover cylinder **3** comes into tight contact with the tapered surface **5e** to block all the urine taking inlets **5b**, **5c**.

In Embodiments 1 and 2, the breaking grooves **48**, **5i** of the blocking members **42b**, **54** may be arcuate grooves. In other words, the blocking members **42b**, **54** may be formed so as to be entirely broken when pushed by a stick for introducing a reagent or a stick for taking out a urine sample.

The blocking members **42b**, **54** may be formed of a blocking ball of different component from the cap **4** or **5** and inserted tightly into the cap **4** or **5**. And, the blocking members **42b**, **54** may be formed so as to be removed from the cap **4** or **5** when pushed by a stick for introducing a reagent or a stick for taking out a urine sample.

The blocking member **42b** may be formed in a thin film and formed with crossed cuts **48a** instead of the breaking groove **48**, as shown in Figure **25**. Namely, the blocking member **42b** functions to block the open end of the vessel body **2** before broken and is broken along the cuts **48a** when pushed by a stick for introducing a reagent for an examination apparatus or a stick for taking out a urine sample.

Needless to say, this construction is applicable to the blocking member **54** in Embodiment 2.

The cuts **48** may be substituted by protrusions.

In other words, the blocking members **42b**, **54** may be of easily breakable construction.

Though the cap body **41** is formed with the knurls **44** in the embodiment 1, the knurls **44** may not necessarily be formed in the present invention.

The cap **4** may not necessarily be formed with the flange section **45** in Embodiment 1 and may be of cylindrical outline.

In the respective embodiments, the shapes of the first urine taking inlet **21**, **5b** and the second urine taking inlet **22**, **5c** are not restricted to ones described therein. Further, the number of the first urine taking inlet **21**, **5b** and the second urine taking inlet **22**, **5c** is not restricted to one for the first urine taking inlet **21**, **5b** and two for the second urine taking inlet **22**, **5c** as described in the embodiments. Furthermore, an opening for examination may be formed.

Though the grip section **53** of the urine taking cap **5** is freely extendable by the extension cylinder **5g** in Embodiment 2, the grip body **5f** itself may be formed telescopically extendably. Alternatively, the grip body **53** may not necessarily be formed extendably.

Though the grip body **53** is formed in a cylinder in Embodiment 2, it may be formed in another shape such as a rectangle.

### Industrial Applicability

As described above, the urine sampling vessel according to the present invention is very useful in taking urine during a urinalysis conducted in hospitals and the like.

## Claims

1. A urine sampling vessel comprising:
an elongated cylindrical vessel body having an end closed and the other end opened;
a urine taking inlet formed in the center of the vessel body;
a cover cylinder, formed into an elongated cylinder having an end closed and the other end opened and with a diameter slightly larger than the vessel body so that the vessel body is freely inserted thereinto and pulled out thereof, for blocking the urine taking inlet;
a cap, formed detachably from the open end of the vessel body, for opening and closing the open end of the vessel body; and
a protrusion for retaining the cover cylinder onto the vessel body at least at a temporarily retained position where the urine taking inlet is opened in a condition that the vessel body is inserted into the cover cylinder.

2. A urine sampling vessel comprising:
an elongated cylindrical vessel body having an end closed and the other end opened;
a urine taking inlet formed in the center of the vessel body;
a cover cylinder, formed into an elongated cylinder having an end closed and the other end opened and with a diameter slightly larger than the vessel body so that the vessel body is freely inserted thereinto and pulled out thereof, for blocking the urine taking inlet;
a cap, formed detachably from the open end of the vessel body, for opening and blocking the open end of the vessel body; and
a breakable blocking member formed in the cap to block the open end of the vessel body.

3. The urine sampling vessel of Claim 2, further comprising
a protrusion for retaining the cover cylinder onto the vessel body at least at a temporarily retained position of the cover cylinder where the urine taking inlet is opened in a condition that the vessel body is inserted into the cover cylinder.

4. The urine sampling vessel of Claim 1 or 3, wherein
the protrusion includes: a plurality of vessel protrusions formed at respective portions of the outer periphery of the vessel body located closer to the closed end of the vessel body than the urine taking inlet and closer to the open end of the vessel body than the urine taking inlet; and a cover protrusion formed in the inner periphery of the cover cylinder to slide over the vessel protrusion when the vessel body is inserted into and pulled out of the cover cylinder, and
the cover protrusion and the corresponding vessel protrusion are constructed to engage each other to retain the cover cylinder onto the vessel body at the temporarily retained position of the cover cylinder where the urine taking inlet is opened and at a full-inserted position of the vessel body where the urine taking inlet is blocked.

5. The urine sampling vessel of Claim 4, wherein
the cap is formed with an engagement groove for engaging the open end of the cover cylinder thereto, and
the cover protrusion and the corresponding vessel protrusion are formed at respective positions where the open end of the cover cylinder is engageable in close contact with the end surface of the engagement groove of the cap so that the cover protrusion slides over the vessel protrusion to make an abutment sound between the cover cylinder and the cap when the vessel body is inserted to its full-inserted position into the cover cylinder.

6. The urine sampling vessel of Claim 4, wherein
the vessel protrusions includes first and second vessel protrusions, disposed at respective portions of the outer periphery of the vessel body closer to the open end thereof than the urine taking inlet and closer to the closed end thereof than the urine taking inlet, for retaining the cover cylinder onto the vessel body at the full-inserted position, and a third vessel protrusion for temporarily retaining the cover cylinder onto the vessel body at the temporarily retained position of the cover cylinder, and
the cover protrusion includes a first cover protrusion, located at a portion of the cover cylinder closer to the open end thereof, for engaging the third vessel protrusion at the temporarily retained position of the cover cylinder and engaging the first vessel protrusion at the full-inserted position of the vessel body, and a second cover protrusion, located at a portion of the cover cylinder closer to the closed end thereof, for engaging the second vessel protrusion at the full-inserted position of the vessel body.

7. The urine sampling vessel of Claim 1, 2 or 3, wherein
a plurality of guide grooves extending in a longitudinal direction of the cover cylinder are formed in a portion of the inner periphery of the cover cylinder located closer to the open end thereof to guidedly introduce urine having been stuck to the surface of the vessel body to the inside of the cover cylinder in inserting the vessel body into the cover cylinder.

8. The urine sampling vessel of Claim 2, wherein
the cap includes a cylindrical cap body, and a blocking plug formed in the cap body so as to be freely press fitted into and pulled out of the open end of the vessel body, and
a retention hole is formed at the other end of the cap body for free insertion and detachment of the cover cylinder, and
the external end wall of the blocking plug is formed as the blocking member.

9. A urine sampling vessel comprising:
a cylindrical vessel body having an end closed and the other end opened for containing taken urine;
a cap which is formed in a hollow cylinder both end surfaces of which are open, has one end formed so as to be fitted in a sealing contact onto the open end of the vessel body and includes a blocking member which partitions an internal space thereof;
a urine taking inlet formed in the center of the cap and located closer to the one end of the cap than the blocking member;
a cover cylinder, formed into a cylinder having an end closed and the other end opened so that the vessel body and the cap are freely inserted thereinto and pulled out thereof, for blocking the urine taking inlet; and
a protrusion for retaining the cover cylinder onto the vessel body at least at a temporarily retained position where the urine taking inlet is opened in a condition that the vessel body is inserted into the cover cylinder.

10. A urine sampling vessel comprising:
a cylindrical vessel body having an end closed and the other end opened for containing taken urine;
a cap which is formed in a hollow cylinder both end surfaces of which are open, has one end formed so as to be fitted in tight contact onto the open end of the vessel body and includes a breakable blocking member which partitions an internal space thereof;
a urine taking inlet formed in the center of the cap and located closer to the one end of the cap than the blocking member; and
a cover cylinder, formed into a cylinder having an end closed and the other end opened so that the vessel body and the cap are freely inserted thereinto and pulled out thereof, for blocking the urine taking inlet.

11. The urine sampling vessel of Claim 10, further comprising
a protrusion for retaining the cover cylinder onto the vessel body at least at a temporarily retained position where the urine taking inlet is opened when the vessel body is inserted into and pulled out of the cover cylinder.

12. The urine sampling vessel of Claim 9 or 11, wherein
the protrusion includes: a vessel protrusion, formed in the outer periphery of the vessel body, for retaining the cover cylinder onto the vessel body at least at the temporarily retained position of the cover cylinder where the urine taking inlet is opened in a condition that the vessel body is inserted into the cover cylinder; and a cover protrusion, formed in the inner periphery of the cover cylinder to slide over the vessel protrusion when the vessel body is inserted into the cover cylinder, for retaining the cover cylinder onto the vessel body by engaging the vessel protrusion at least at the temporarily retained position of the cover cylinder.

13. The urine sampling vessel of Claim 12, wherein
the cap is formed with a radially enlarged annular member engageable with the open end of the cover cylinder, and
the vessel protrusion and the cover protrusion are formed at respective positions where the open end of the cover cylinder is engageable in close contact with the annular member of the cap so that the cover protrusion slides over the vessel protrusion to make an abutment sound between the cover cylinder and the cap when the vessel body is inserted to its full-inserted position into the cover cylinder.

14. The urine sampling vessel of Claim 12, wherein
the cover protrusion includes: a first cover protrusion, located at a portion of the cover cylinder closer to the open end thereof, for engaging the vessel protrusion at the temporarily retained position of the cover cylinder; and a second cover protrusion, located at a portion of the cover cylinder closer to the closed end thereof, for engaging the vessel protrusion at the full-inserted position of the vessel body where the urine taking inlet is blocked.

15. The urine sampling vessel of Claim 9, 10 or 11, wherein
a plurality of guide grooves extending in a longitudinal direction of the cover cylinder are formed in a portion of the inner periphery of the cover cylinder located closer to the open end thereof to guidedly introduce urine having been stuck to the surfaces of both the vessel body and the cap to the inside of the cover cylinder in inserting the vessel body into the cover cylinder.

16. The urine sampling vessel of Claim 9, wherein
the vessel body is constructed so as to be set in an examination apparatus in a condition that the cap is detached with the vessel body held inserted into the cover cylinder or a condition that both the cap and the cover cylinder are detached.

17. The urine sampling vessel of Claim 10, wherein
the vessel body is constructed so as to be set in an examination apparatus with the cap held attached thereto and inserted into the cover cylinder.
